# EUROPEAN PATENT SPECIFICATION

(11) **EP 1 909 915 B1**
(45) Date of publication and mention of the grant of the patent: **31.03.2010**
(21) Application number: 06800651.9
(22) Date of filing: 02.08.2006
(51) Int. Cl.: A61Q 11/00, G01N 33/497

(54) **SCREENING METHOD FOR THE IDENTIFICATION OF COMPOSITIONS SUITABLE FOR THE TREATMENT OF ORAL CAVITY MALODOR ASSOCIATED WITH SMOKING A TOBACCO PRODUCT**
SCREENING-VERFAHREN ZUR IDENTIFIZIERUNG VON ZUSAMMENSETZUNGEN FÜR DIE BEHANLDUNG VON MUNDGERUCH INFOLGE DES RAUCHENS EINES TABAKPRODUKTS
METHODE DE CRIBLAGE POUR L'IDENTIFICATION DE COMPOSITIONS DESTINEES A COMBATTRE LES MAUVAISES ODEURS DE LA CAVITE BUCCALE ASSOCIEES AU TABAC

(30) Priority: 02.08.2005 US 704597 P
(43) Date of publication of application: 16.04.2008
(73) Proprietor: WM. WRIGLEY JR. COMPANY, Chicago, Illinois 60611-4287 (US)
(72) Inventor: BAZEMORE, Russell, Albert, Grant, AL 35747 (US); HARRISON, Charles, J., Ann Arbor, MI 48105 (US)
(74) Representative: Hayes, Adrian Chetwynd
(86) International application number: PCT/US2006/030069
(87) International publication number: WO 2007/016619

(56) References cited:
- WO-A-03/105794
- WO-A2-2005/048965
- US-A- 2 091 497
- US-A- 4 626 427
- US-A- 4 775 537
- BAZEMORE RUSSELL ET AL: "Identification of components responsible for the odor of cigar smoker's breath." JOURNAL OF AGRICULTURAL AND FOOD CHEMISTRY. 25 JAN 2006, vol. 54, no. 2, 25 January 2006 (2006-01-25), pages 497-501, XP002413765 ISSN: 0021-8561

## Description

### FIELD OF THE INVENTION

This invention generally relates to a screening method for identifying compositions suitable for use in an oral composition (e.g., a confection or chewing gum product) effective for the treatment of oral cavity malodor associated with smoking a tobacco product. In particular, this invention relates to a screening method for determining the ability of a composition to reduce the concentration of a pyridine or pyrazine compound present in a model sample or solution which is representative of the oral cavity of a subject after smoking a tobacco product, as an indicator of the effectiveness of that composition in the treatment of oral malodor associated with smoking a tobacco product.

### BACKGROUND OF THE INVENTION

It has been estimated that more than 1.2 billion people worldwide smoke tobacco products. It is known that pyrolyzed tobacco often results in a lingering odor in the oral cavity of persons who smoke tobacco products. In addition, it is readily apparent to smokers and their associates that pyrolyzed tobacco volatiles are released from the smoker's oral cavity, air passageways and lungs and are present in the smoker's breath in sufficient quantity to be perceived by others. These odors are also generally perceived as aftertaste by the smoker, but in a manner consistent with the concept of odor adaptation; that is, constant exposure to the odor decreases perception over time such that the smoker may over time become immune to at least one of the indicators of the unpleasant odor which may be perceived by others.

The widespread use of tobacco products and oral malodor associated therewith has created a consistent demand for breath freshening products. However, it has been estimated that approximately 4,800 compounds may be generated upon pyrolysis of tobacco. Furthermore, smoking the tobacco product also results in pyrolysis of additives found in cigarettes, and up to about 600 different additives may be utilized in cigarettes.

Successful strategies for the amelioration of oral malodour associated with tobacco smoke have to-date been difficult to develop, at least in part due to the myriad possible sources of oral malodour. In addition, in order for a substance to possess aroma, it typically is volatile and passes through a person's nasal epithelium retronasally (i.e. through the mouth) or orthonasally (i.e. by sniffing).
US-A-4,626,427 describes preparations being effective, when chewed and swallowed, against bad breath, originating from the consumption of smelling substances, including onions, garlic, alcohol and smoking tobacco, **characterized in that** it comprises a chewing bonbon containing uncoated seeds of the cardamom plant, or cardamom seeds coated by a dragee shell mass, said chewing bon bon and said dragee shell mass consisting essentially of sugar or sugar substitutes including sorbitol, xylitol and mixtures thereof.
WO-A-2005/048965 discloses an oral composition for freshening the breath of consumers of tobacco products which comprises an oral cavity delivery agent and an effective amount of a polyphenolic apple extract.
US-B-4,775,537 describes a chewing gum composition which comprises a gum base, one or more non-confined water soluble flavouring agents, one or more non-confined oil soluble flavouring agents and optionally, conventional chewing gum additives. Cardamom flavour is an example of an oil soluble flavouring agent.

### SUMMARY OF THE INVENTION

Briefly, therefore, the present invention is directed to a method for identifying a composition suitable for use in an oral composition effective for reducing oral malodour associated with tobacco smoke, the method comprising:
contacting in a vessel a test composition and a model solution comprising tobacco smoke odorants, the odorants comprising pyridine, 2-ethyl pyridine, 3-ethyl pyrazine and ethyl pyrazine, wherein the test composition comprises cardamom oil, rosemary extract, basil extract, thyme extract, parsley seed oil, zinc lactate, eugenol, α-cyclodextrin, β-cyclodextrin, γ-cyclodextrin, activated carbon, or a combination thereof; and
determining the ability of the test composition to reduce the concentration of one or more of the odorants in said model solution in the headspace of said vessel.

In a preferred embodiment, the method comprises contacting in a vessel a test composition and a model solution comprising tobacco smoke odorants, the odorants comprising pyridine, 2-ethyl pyridine, 3-ethyl pyrazine and ethyl pyrazine, and determining the ability of the test composition to reduce the concentration of one or more of the odorants in the model solution in the headspace of the vessel.

In a further preferred embodiment, the method comprises preparing a plurality of test compositions, individually contacting in a vessel each of the test compositions and a model solution comprising a pyridine or pyrazine compound present in tobacco smoke, determining the ability of each of the test compositions to reduce the concentration of the pyridine or pyrazine compound in the headspace of each of the vessels and identifying one or more test compositions as effective to reduce the concentration of the pyridine or pyrazine compound by at least 50%.

The present invention is also directed to the use of a chewing gum comprising cardamom oil, cranberry extract, or a combination thereof to reduce the concentration of a pyridine or pyrazine compound present in a subject's oral cavity as a result of smoking a tobacco product.

In a preferred embodiment the concentration of the pyridine or pyrazine compound is reduced by at least 50%.

### BRIEF DESCRIPTION OF THE DRAWINGS

Fig. 1 is a flame ionization detector (FID) chromatogram prepared as described in Example 1.

Fig. 2 is a total ion current (TIC) chromatogram prepared as described in Example 1.

Fig. 3 is an overlay of an aromagram and TIC chromatogram described in Example 1.

Fig. 4 shows the headspace concentration change of odorous components following treatment of a model solution with Applephenon^{®} as described in Example 2.

Fig. 5 shows the results of sensory analysis of model solutions treated with Applephenon^{®} as described in Example 2; error bars indicate standard error.

Fig. 6 shows the percent reduction in headspace concentration of various components following treatment of a model solution with Applephenon^{®} as described in Example 3.

Fig. 7 shows sensory analysis results comparing aftertaste intensity for subjects after smoking cigarettes and after chewing a control gum and a gum containing added active (Applephenon^{®}) as described in Example 4.

Fig. 8 shows sensory analysis results comparing aftertaste intensity for subjects after smoking cigars and after chewing a control gum and a gum containing added active (Applephenon^{®}) as described in Example 5.

Fig. 9 shows cranberry extract efficacy for reducing headspace concentration of odorants from a model solution headspace as described in Example 6; error bars indicate standard error.

Fig. 10 shows cardamom oil efficacy for reducing headspace concentration of odorants from a model solution headspace as described in Example 6; error bars indicate standard error.

Fig. 11 shows the effect of cranberry extract in reducing odor from pyridine and pyrazine components of a tobacco smoke model solution in terms of percent decrease vs. control as described in Example 6; error bars indicate standard error and sensory analysis was conducted utilizing a 0-10 point line scale.

Fig. 12 shows the effect of cardamom oil in reducing odor from pyridine and pyrazine components of a tobacco smoke model solution in terms of percent decrease vs. control as described in Example 6; error bars indicate standard error and sensory analysis was conducted utilizing a 0-10 point line scale.

Fig. 13 shows cardamom oil release (percent cardamom over time) as described in Example 7.

Fig. 14 shows cardamom oil release (amount of cardamom over time) as described in Example 8.

### DETAILED DESCRIPTION OF THE PREFERRED EMBODIMENTS

In accordance with the present invention, and as further detailed herein below, it has been discovered that contributors to oral cavity malodor associated with smoking a tobacco product, such as pyridine and/or pyrazine compounds, may be used to prepare model samples or solutions for the purpose of evaluating or screening potential active or test compositions (e.g., cardamom oil) for their ability or effectiveness in reducing the concentration of these indicator or target compounds in the gaseous atmosphere, or headspace, of a vessel in which they are contained. It has been still further discovered that the ability or effectiveness of an active or test composition to reduce the concentration of these indicator compounds in the headspace of the vessel is also indicative of the ability or effectiveness of that composition to reduce the concentration of these volatile, odor-causing compounds that are present in a subject's oral cavity after smoking a tobacco product. Accordingly, such an active or test composition may be well-suited for incorporation into an oral composition including, but not limited to, a confection, chewing gum, lozenge, pressed tablet, edible film, mouthspray, mouthwash, or toothpaste product suitable for treatment of oral cavity malodor associated with smoking a tobacco product.

### Odor-causing Compounds

Various aromatic nitrogenous compounds, including pyridine and/or pyrazine compounds, have been discovered to contribute to the oral malodor associated with smoking a tobacco product. In particular, it has been discovered that di- and tri-substituted pyridine and/or pyrazine compounds, including for example lower alkyl (e.g., C₁, C₂, C₃, C₄) substituted pyridine and/or pyrazine compounds (such as methyl, ethyl, propyl, butyl and/or cyclobutyl, di- or tri-substituted pyridine and/or pyrazine compounds), contribute to this oral malodor. Without being held to a particular theory, it is presently believed that the various pyridine and pyrazine compounds may be by-products of nicotine pyrolysis; in particular, in the case of pyridine compounds, pyrolysis is believed to result in cleavage of the bond between carbon number 2 and number 3 in the pyrrolidine moiety of nicotine (i.e., 3-(1-methyl-2-pyrrolidinyl) pyridine, illustrated below).

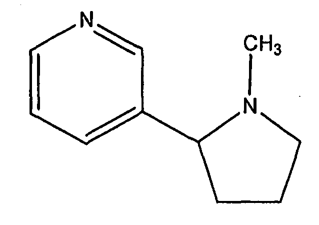

In addition to the various pyridine and pyrazine compounds present, a pyrrole (e.g., ethyl pyrrole) has also been identified and linked to tobacco smoke malodor. Non-nitrogenous compounds, such as acetophenone (i.e., 1-phenylethanone) and diacetyl (i.e., 2,3-butanedione), have been identified and linked to tobacco smoke malodor, as well.

Exemplary odorous components responsible for oral malodor caused by smoking a tobacco product identified in accordance with the present invention are listed in Table 1, provided below in Example 1. Certain of these odor-generating substances present in the smoke of tobacco products may be present at extremely low levels (e.g., parts per million or parts per billion levels), yet still contribute to noticeable odor. For example, pyridine has been reported to have an odor threshold value in water of 840 parts per billion (Flavor Base software, Leffingwell and Associates, Canton, GA, 2004).

### Techniques for Identifying Odor-causing Compounds

In accordance with the present invention, techniques known in the art were utilized to identify the above-noted compounds as a primary source of odor in a subject's oral cavity, after smoking a tobacco product. Generally speaking, odorous tobacco smoke compounds may be extracted from the oral cavity of a subject who has smoked a tobacco product using various techniques known in the art, including for example swabbing the subject's tongue to provide a sample to be analyzed to determine the particular compounds contributing to the malodor. Verification of the presence of tobacco smoke odorants on the swab is typically carried out by trained odor judges, who smell the swabs. Odorous tobacco smoke compounds may additionally, or alternatively, be collected by taking breath samples from the oral cavity of a subject who has smoked a tobacco product, also using various techniques known in the art. For example, the subject may exhale into a vessel containing simulated saliva (e.g., a solution containing sodium chloride, sodium bicarbonate and potassium bicarbonate in deionized water, prepared as described below in Example 1) in order to collect a sample.

Once collected, the odor-causing compounds present in these samples which contribute to the oral malodor associated with smoking a tobacco product may be identified using a variety of techniques known in the art. For example, the headspace of an airtight or hermetically sealed vessel in which the substances are contained (e.g., a vessel containing a swab taken from a smoker's oral cavity or a portion of a liquid sample containing tobacco smoke odorants (e.g., simulated saliva)) may be isolated by solid phase microextraction (SPME) and subjected to further analysis (e.g., gas chromatography, used in combination with a device suitable for compound detection or identification, such as a mass spectrometer) to determine the individual compounds contributing to the malodor.

Solid phase microextraction is a well-known method suitable for extracting a sample containing odorous components from a subject's oral cavity for subsequent analysis. (See, for example, *Released Oral Malodors Measured by Solid Phase Microextraction*/*Gas Chromatography Mass Spectrometry (HS-SPME-GC-MS),* Payne, R., Labows, J., Liu, X, Proceeding of ACS - Flavor Release No. 0841236925, 2000.) For example, hydrogen sulfide, methyl mercaptan and dimethyl sulfide have been detected by extraction of mouth air with a gas tight syringe followed by separation with a packed column and detection with a flame photometric detector. (See, for example, Direct Gas Chromatograph Analysis of Sulphur Compounds in Mouth Air in Man, Tonzetich, J., Archs, Oral Biol. 1971, 16, 587-597.) In particular, SPME has proven to be suitable for extracting volatile components from the headspace of vessels containing unconventional odorous substances for subsequent analysis for odor by gas chromatography-olfactometry (GCO) analysis and gas chromatography-mass spectrometry (GC/MS) analysis. (See, for example, Headspace Solid Phase Microextraction, Zhang, Z., Pawliszyn, J., Anal. Chem. 1993, 65, 1843-1852.) Benefits of SPME include few requirements with respect to sample preparation, little need for solvent, and relatively fast extraction times. Suitable apparatus for the GC/MS analysis include, for example, an Agilent 6890 gas chromatograph (GC) / mass spectrometer (MS) available from Agilent Technologies (Palo Alto, CA).

In one approach, a sample known to contain odorants may be analyzed using a gas chromatograph equipped with a mass spectrometer. Identification of the odor-causing components present therein may then generally be performed by matching sample spectra with a database (e.g., Wiley Registry of Mass Spectral Data, 7th Edition, John Wiley & Sons, Inc., 2000) and/or matching retention indices of components of the sample with known standards.

In an alternative approach, identification of the odorants may be conducted by gas chromatography-olfactometry (GCO) analysis, which comprises determining which portions of the GC column eluant exhibit tobacco smoke odor characteristics utilizing a sniff port and then subjecting those portions of the eluant to further analysis (e.g., mass spectrometry) to determine their composition. Various GCO techniques have been described in literature. One such method includes the Osme method, described in Odor analysis of Pinot Noir wines from Grapes of Different Maturities by a Gas Chromatography-Olfactometry Technique (Osme), Miranda-Lopez, R., Libbey, L.M., Watson, B.T., McDaniel, M.R., J. Food Sci., 1992, 57: 985-993, 1019. Another method includes CHARM analysis, described in A procedure for the sensory analysis of gas chromatographic effluents, Acree, T. E.; Barnard, J.; Cunningham, D. G, Food Chem. 1984, 41, 1698-1703. Still another method includes aroma extraction dilution analysis, described in Characterization of saffron flavor by aroma extract dilution analysis, Cadwallader, K. R.; Baek, H. H.; Cai, M, Spices; Shahidi, F.; Cadwallader, K. R., Eds.; American Chemical Society: Washington, DC, 1997, 66-79.

### Model Solutions

In accordance with the present invention, once identified, the above-noted odor-causing compounds may be used to prepare solutions which model or mimic saliva present in the oral cavity of a subject who has smoked a tobacco product. These model solutions may be used to determine the efficacy of potential active compositions (e.g., test compositions) for ameliorating oral malodor attributed to smoking a tobacco product; that is, these model solutions may be used to determine the ability of a test composition to reduce the concentration of one or more of the odor causing compounds present in the gaseous atmosphere, or headspace, of a vessel in which the model solution is contained, which is in turn an indicator of the ability of the test composition to achieve a similar result in the oral cavity.

The similarity between the odor character and intensity of the model solution and the breath of a subject who has smoked a tobacco product may be determined by trained odor judges. In general, a model solution is prepared by adding one or more of the identified tobacco smoke odorant compounds to a liquid (e.g., aqueous) medium or solvent. The model solution may also be prepared by adding a liquid medium or solvent to a vessel containing one or more tobacco smoke odorant compounds. The liquid medium typically comprises water, and may optionally comprise one or more additional components (e.g., an alcohol, such as ethanol or methanol). In various embodiments, the aqueous medium to which the tobacco smoke odorant compounds are added may comprise a combination of alcohol (e.g., ethanol) and water, at various concentrations or ratios. For example, a suitable aqueous medium typically contains from 1% to 20% (by weight) or from 1% to 10% (by weight) of an alcohol such as ethanol, and from 80% to 99% (by weight) water (e.g., a 5% ethanol/95% water solution (by weight) or a 1% ethanol/99% (by weight) water solution).

Generally, the model solution is prepared to assure, in the vessel in which it is contained, an initial and final odorant headspace concentration which exceeds the analysis method detection limit sufficiently such that errors in detection/measurement are minimized or avoided. Thus, typically, the model solution is prepared to provide a minimum headspace concentration of odorants in the vessel of at least 10 parts per million (ppm), at least 50 ppm, at least 100 ppm, at least 150 ppm, or at least 250 ppm.

The model solution typically contains one or more tobacco smoke odorants such that the solution has a total odorant concentration of at least 200 parts per million (ppm) (i.e., micrograms of odorant per milliliter solution), more typically at least 600 ppm, still more typically at least 1000 ppm and, still more typically, a total odorant concentration of at least 1500 ppm. Preferably, the odorant or odorants are present in the model solution at a total concentration of from 600 to 2000 ppm, more preferably at a total concentration of from 600 to 1750 ppm and, more preferably, at a total concentration of from 1000 to 1750 ppm. The liquid (e.g., aqueous) medium or solvent (e.g., water, and optionally an alcohol or some other component) typically makes up the remaining portion of the model solution.

Typically, the model solution comprises a pyridine or pyrazine compound and, in various embodiments, the model solution comprises both a pyridine compound and a pyrazine compound. In one preferred embodiment, the solution comprises a plurality of pyridine compounds and/or a plurality of pyrazine compounds. The pyridine compound(s) may be selected from, for example, 2,4,6-trimethyl pyridine, 2,6-dimethyl pyridine, 3,5-dimethyl pyridine, 2-ethyl pyridine, 3-ethyl pyridine and pyridine, or some combination thereof. The pyrazine compound(s) may be selected from ethyl pyrazine, 2,3-dimethyl pyrazine, 2,5-dimethyl pyrazine and 2-ethyl-3-methyl pyrazine, or some combination thereof. For example, in at least some embodiments, the model solution comprises 2,4,6-trimethyl pyridine, 2,6-dimethyl pyridine, 2-ethyl pyridine, 3-ethyl pyridine, pyridine, ethyl pyrazine, and/or 2-ethyl-3-methyl pyrazine. In still further embodiments, the model solution additionally or alternatively comprises pyridine, 2-ethyl pyridine, 3-ethyl pyrazine and/or ethyl pyrazine.

Additionally, it is to be noted that, optionally, one or more model solutions may also contain a pyrrole (e.g., ethyl pyrrole) and/or non-nitrogenous compounds such as acetophenone (i.e., 1-phenylethanone) and diacetyl (i.e., 2,3-butanedione).

In accordance with the present invention, various model solutions which are particularly representative of or similar to tobacco smoke odor have been identified. One such solution comprises pyridine, 2-ethyl pyridine, 3-ethyl pyrazine and ethyl pyrazine. Optionally, the model solution may additionally comprise these components as well as 2,4,6-trimethyl pyridine, 2,6-dimethyl pyridine, 3-ethyl pyridine, 2-ethyl-3-methyl pyrazine, or combinations thereof. These model solutions may optionally further comprise 3,5-dimethyl pyridine, 2-ethyl pyridine, 3-ethyl pyridine, 2,3-dimethyl pyrazine, 2,5-dimethyl pyrazine, or combinations thereof.

In the case of one or more of the model solutions described above and/or elsewhere herein (e.g., a solution comprising pyridine, 2-ethyl pyridine, 3-ethyl pyrazine and ethyl pyrazine), the total odorant concentration is typically at least 200 ppm, and preferably is at least 600 ppm, and more preferably is at least 1000 ppm, the concentration ranging for example from 200 to 1200 ppm, or from 200 to 800 ppm. By way of further example, in the case of a model solution which comprises pyridine, 2-ethyl pyridine, 3-ethyl pyrazine, ethyl pyrazine, 2,4,6-trimethyl pyridine, 2,6-dimethyl pyridine, 3-ethyl pyridine and 2-ethyl-3-methyl pyrazine, the total odorant concentration is typically at least 400 ppm, and preferably is at least 800 ppm, and more preferably is at least 1000 ppm, the concentration ranging for example from 400 to 1400 ppm, or from 800 to 1400 ppm. More particularly, for one or more of the model solutions described herein above and/or elsewhere herein, each of the odorants may be present in the model solution at a concentration of 1 ppm, of at least 50 ppm, preferably of at least 75 ppm, and more preferably of at least 100 ppm, the concentration of each odorant ranging for example from 1 to 200 ppm, from 125 to 175 ppm, or from 75 to 150 ppm.

### Screening Method

In accordance with the present invention, the above-noted model solution may be utilized as part of a method for screening a composition to determine whether that composition is effective for reducing odorant concentration in the gaseous atmosphere or headspace of a vessel in which a model solution is contained. The vessel in which the model solution is contained is generally of a size appropriate to provide the desired minimum odorant headspace concentration of the vessel in which the odorants are contained described elsewhere herein. In general, the screening method of the present invention comprises contacting the model solution and a test composition. Generally, the test composition may be in the form of an oil (e.g., cardamom oil), a solution of the test composition in an aqueous medium (e.g., water) or in the form of a solid which is dissolved upon contact with the model solution. The ability of the test composition to reduce the concentration of a pyridine or pyrazine compound in the headspace of the vessel in which the model solution containing the odorant(s) is contained may be determined using techniques known in the art. More specifically, this determination may be made by, for example, measuring the concentration of the pyridine or pyrazine compound in the headspace of the vessel containing the solution prior to contact with the test composition, and then measuring the concentration of the pyridine or pyrazine compound in the headspace of the vessel containing the solution after contact with the test composition. The difference between the initial and final concentration of the pyridine compound(s) and/or pyrazine compound(s) in the headspace of the vessel thus indicates the effectiveness of the test composition for reducing the odorant headspace concentration.

In this regard it is to be noted that, as further detailed elsewhere herein, there has been observed to be a correlation between a quantitative determination of effectiveness of a test composition for reducing odorant headspace concentration and the qualitative performance of an oral composition containing such a composition for treating oral cavity malodor associated with smoking a tobacco product.

Both the initial and final pyridine and/or pyrazine concentrations in the headspace of the vessel are generally determined using means known in the art including, as detailed elsewhere herein, by taking a sample of the headspace and subjecting the sample portion of the vapors to analysis comprising separation, such as by chromatography, and detection; such as by mass spectrometry. Sampling of the headspace may be conducted by contacting the headspace with a fiber effective for absorbing a portion of the vapors comprising the pyridine or pyrazine compound in the headspace, or by contacting the headspace with a gas tight syringe effective for extracting a portion of the vapors comprising the pyridine or pyrazine compound in the headspace. Sampling or extracting a portion of the headspace is typically conducted at a temperature of from 20°C to 100°C or from 20°C to 40°C, preferably from 20 to 25°C and, still more preferably, at a temperature of about 22°C. In addition, the sampling or extracting of the headspace typically proceeds for at least 5 minutes, at least 10 minutes, at least 20 minutes, at least 30 minutes, or at least 1 hour. Generally, sampling or extraction proceeds over a period of up to 2 hours, up to 3 hours, up to 4 hours, up to 6 hours, up to 8 hours, or up to 10 hours. In various other embodiments, sampling and extraction may proceed over the course of significantly longer periods of time (e.g., up to 12 hours, up to 24 hours, or up to 48 hours). Multiple samples of the headspace may be taken consecutively and, in fact, multiple samples may be extracted during one or more of the sample times set forth above. Additionally or alternatively, samples may be taken intermittently in accordance with the sample times set forth above, with the interval between samplings not narrowly critical.

In this regard it is to be noted that sample analysis and the determination of the concentration of a given odor causing compound in the vessel headspace, either before or after contact with a test composition, may be performed using other techniques or methodologies known in the art without departing from the scope of the present invention.

The model solution and the test composition are typically contacted in an airtight or hermetically sealed vessel. To ensure sufficient contact between the test composition and the model solution, the test composition and the model solution are typically contacted for at least 5 minutes, at least 10 minutes, at least 20 minutes, at least 30 minutes, or at least 1 hour prior to determining the final pyridine and/or pyrazine concentration. Generally, the test composition and the model solution are contacted for a period of up to 2 hours, up to 3 hours, up to 4 hours, up to 6 hours, up to 8 hours, or up to 10 hours, prior to determining the final pyridine and/or pyrazine concentration. In various embodiments, the test composition and the model solution are contacted for significantly longer periods of time (e.g., up to 12 hours, up to 24 hours, or up to 48 hours).

A suitable temperature for contacting the test composition and model solution is selected in order to preferably simulate conditions of the oral cavity of a subject consuming an oral composition (e.g., chewing a gum or consuming a confection), in which the test composition would be used. Thus, typically, the test composition and the model solution are contacted at a temperature of from 20 to 100°C, from 20°C to 40°C or from 20°C to 30°C, and preferably from 20°C to 25°C and, still more preferably, at a temperature of about 22°C.

The vessel containing the test composition and model solution may also be agitated, for example to simulate consumption (e.g., chewing) conditions. The degree and manner of agitation are not narrowly critical and may be conducted in accordance with methods known in the art.

Typically, the model solution is contacted with a quantity of the test composition that is representative of the amount of the test composition that would ultimately be used in an oral composition such as a chewing gum or confection product. Since the proportion of test composition incorporated into an oral composition may vary depending on, for example, the type of composition in which it is incorporated and the desired flavor of the composition, the quantity of test composition contacted with the model solution may likewise vary within relatively broad limits. For example, the model solution may in some embodiments of the present invention be contacted with at least 0.01 milligram (mg) of the test composition per milliliter (ml) of solution, at least 0.1 mg of the test composition per ml of solution, at least 0.5 mg of the test composition per ml of solution, or at least 1 mg of the test composition per ml of solution. In at least some embodiments, the model solution is contacted with from 0.01 to 1 mg of the test composition per ml of solution, from 0.1 to 0.8 mg of the test composition per ml of solution, or from 0.2 to 0.7 mg of the test composition per ml of solution. In various other embodiments, the model solution is typically contacted with from 1 to 75 mg of the test composition per ml of solution, more typically from 5 to 60 mg of the test composition per ml of solution, preferably from 10 to 50 mg of the test composition per ml of solution and, more preferably, from 15 to 30 mg of the test composition per ml of solution.

It is to be noted that the screening method of the present invention is amenable to testing a plurality of compositions using known combinatorial techniques. In such embodiments, a plurality of test compositions (e.g., a library or an array of, for example, at least 5, at least 10, at least 15, at least 20, at least 25, at least 30, at least 40, at least 50 or more test compositions) may be prepared and contacted with the same, or a different, model solution in, for example, individual hermetically sealed vessels, or alternatively in individual hermetically sealed wells of a common substrate. Preferably, the plurality of test compositions are arranged in a spatially addressable format, such as in wells of a common substrate in a spatially addressable format (e.g., a microtiter plate), to enable the present method to be more easily carried out using commercially available automation (e.g., commercially available auto-sampling devices that may be used in combination with, for example, a commercially available GC/MS device). Advantageously, the ability of each of the plurality of test compositions to reduce the concentration of a pyridine or pyrazine compound in each of the vessels may be determined in parallel. In at least certain embodiments, an oral composition (e.g., confection or chewing gum) is prepared from at least 2 or more of the plurality of test compositions thus identified as effective for reducing pyridine and/or pyrazine concentrations.

Without being bound by a particular theory, reduction of the headspace concentration of a pyridine and/or a pyrazine compound attributed to contact with the test compositions as described herein is believed to proceed in accordance with one or more mechanisms.

One possible mechanism is the effect of addition of the test composition on the pH of the model solution. For example, model solutions containing pyridine and/or pyrazine compounds are typically neutral (i.e., have a pH of from 6 to 8). A drop in pH (i.e., acidifying the solution) was observed upon addition of various test compositions to such model solutions. For example, addition of Applephenon^{®} to a model solution (as detailed in Example 2) produced an acidic solution having a pH of approximately 3.7. This drop in pH is presently believed to be attributed to a major component of Applephenon^{®}, the acidic polyphenol chlorogenic acid. In an acidified solution containing pyridine, pyridine (which has a pKa of 5.2) exists primarily as a cation. It is presently believed that hydrogen bonding of the charged pyridine species results in a protonated form having decreased volatility and, accordingly, reduced concentration in the headspace. In addition, a charged component of a model solution (positive or negative) is typically more water soluble than an uncharged species. An increase in solubility of odorous components is typically associated with greater salivary flow in the oral cavity, which can result in increased removal of the odorants from the oral cavity by swallowing prompted by the increased salivary flow. Removal of the soluble components from the oral cavity may also proceed by rinsing with water or other beverages.

In view of the foregoing, it is therefore to be noted that, in some embodiments, the screening method of the present invention may additionally, or alternatively, involve the screening of the same test composition and/or model solution, but at differing pH values, in order to evaluate the impact of pH on the performance of the test composition. For example, in such an embodiment, the contents of the test composition may be controlled so that, when contacted with the model solution, the resulting mixture or solution has a pH value of less than 7, ranging for example from 2 to 6.

Another possible mechanism involves the effect of components of certain active compositions on salivary protein production. Polyphenols are components of certain of the active compositions (e.g., Applephenon^{®} and cranberry extract). It is known that polyphenols, particularly tannins, precipitate salivary proteins. It is presently believed that volatile components adsorb to salivary proteins making them less likely to be released into the headspace of the solution. Also, a portion of the volatile components are believed to be removed from the oral cavity by virtue of swallowing of the salivary proteins having volatile components adsorbed thereto.

Still another possible mechanism involves a reaction between a component of the active and an odor-causing compound. More particularly, a reaction between polyphenol (from a test composition such as cranberry extract) and nitrogenous compounds from tobacco smoke (e.g., pyridines), thereby reducing the concentration of these volatile components. Another active composition of the present invention, cardamom, contains terpenes, most notably limonene. These compounds possess antioxidant activity in a manner similar to polyphenols. Thus, it is presently believed that there may be a similar reaction between the terpenes associated with cardamom and pyridines and an attendant reduction in concentration of the volatile component.

Regardless of the mechanism by which the concentration reduction is achieved, it is to be noted that the present invention enables the screening of test compositions in a quantifiable, and/or analytical, way, in order to evaluate their potential use in an oral composition such as a chewing gum or confection product, without having to initially prepare such an oral composition. As such, a plurality of samples may be evaluated more rapidly and in a more efficient and cost effective manner. For example, in a preferred embodiment, the present invention may be utilized to screen a plurality of test compositions in order to more efficiently identify and select test compositions that are effective to reduce the concentration of one or more pyridine and/or pyrazine compounds in the headspace of a vessel containing a model solution (e.g., as determined by mass spectrometry using means known in the art) by at least 20% or at least 30%, preferably at least 40%, more preferably at least 50%, still more preferably at least 60%, still more preferably at least 70%, still more preferably at least 80%, still more preferably at least 90%, and most preferably 100%. Once identified, these compositions may then optionally be subjected to further testing, wherein they are formulated into an oral composition for further testing. In some instances, these resulting compositions (e.g., gums and/or confections) may achieve the same or similar reduction in the oral cavity of a test subject.

As previously noted, in order for a substance to possess aroma, it typically is volatile and passes through a person's nasal epithelium retronasally or orthonasally. Thus, reduction in headspace concentration of the volatile odorants of a model solution generally indicates effectiveness for reduction of volatile components present in a subject's oral cavity after smoking a tobacco product and, accordingly, treatment of oral cavity malodor caused by the presence of these volatile components. Accordingly, once a test composition has been successfully identified in accordance with the present invention to reduce the concentration of an odor causing compound, this test composition may then optionally be used to prepare an oral composition (e.g., chewing gum and/or confection product) using means known in the art, for further evaluation or use with human subjects.

Various compositions may be screened using the process described herein to determine their ability to reduce the odorant headspace concentration of model solutions, including compositions derived from various fruits (e.g., cranberries, apples, crabapple, hawthorn berries, plums, prunes and grapes), vegetables, and plants.

For example, compositions to be screened in accordance with the present invention may comprise, or alternatively consist essentially of, an extract, oil, compound, etc. selected from the group consisting of cardamom seed extract (e.g., cardamom oil), rosemary extract, basil extract, thyme extract parsley seed oil, zinc lactate, eugenol, α-cyclodextrin, β-cyclodextrin, γ-cyclodextrin, and combinations thereof.

By way of further example, compositions to be screened in accordance with the present invention may comprise, or alternatively consist essentially of, an extract, oil, compound, etc. selected from the group consisting of cardamom seed extract (e.g., cardamom oil), rosemary extract, basil extract, thyme extract parsley seed oil, zinc lactate, eugenol, α-cyclodextrin, β-cyclodextrin, γ-cyclodextrin, and combinations thereof.

In still further embodiments, the composition to be screened may be derived from a plant including, for example, cardamom seed extract (e.g., cardamom oil) rosemary extract, basil extract, thyme extract, parsley seed oil and combinations thereof. In still further embodiments, the composition to be screened comprises zinc lactate, eugenol, α-cyclodextrin, β-cyclodextrin, γ-cyclodextrin and combinations thereof.

Carbon (e.g., activated carbon) obtained from, for example, wood or nutshells may also be screened in accordance with the present invention. In particular, cardamom oil has been determined to be effective for reducing odorant headspace concentration.

### Use of Compositions in Oral Compositions

Compositions recognized as effective to reduce the concentration of a pyridine or pyrazine compound in the headspace of the vessel in which the substances are contained by at least 50% (or greater) are particularly well-suited for incorporation into an oral composition including, but not limited to, a confection, chewing gum, lozenge, pressed tablet, edible film, mouthspray, mouthwash, or toothpaste product suitable for treatment of oral cavity malodor associated with smoking a tobacco product. In particular, a test, or an active, composition identified as effective for reducing the concentration of an odor causing compound in the headspace of a vessel in which the compound is contained, in accordance with the present screening method, is suitable for incorporation into a confection or chewing gum in accordance with methods known in the art as described, for example, in U.S. Patent No. 6,627,234.

The active composition may be incorporated into an oral composition without dilution, or it may be diluted prior to incorporation. In either case, the active composition may be present in a confection or chewing gum, for example, at a concentration of at least 0.1% by weight, more typically at least 0.5% by weight and, still more typically, 1% by weight. Preferably, the active composition is present in the confection or chewing gum at a concentration of from 0.1% to 5% by weight, more preferably from 0.5% to 2% by weight and, still more preferably, at a concentration of from 0.5% to 1% by weight.

### Method for Treating Oral Malodor

Generally, treatment of oral malodor associated with tobacco smoke proceeds by administration to a subject an oral composition (e.g., one or more pieces of a confection or chewing gum product) containing an active composition identified in accordance with the present invention; that is, oral malodor may be treated in accordance with this invention by administering an oral composition recognized to reduce the concentration of tobacco smoke odorants in a subject's oral cavity. More particularly, oral malodor may be treated by administering to a subject a composition effective to reduce the concentration of a pyridine or pyrazine compound present in the subject's oral cavity as a result of smoking a tobacco product by at least 20%, at least 30%, at least 40%, at least 50%, at least 60%, at least 70%, at least 80%, or at least 90%. For example, in the case of an oral composition such as a chewing gum or confection, administration may comprise chewing multiple pieces of the oral composition. It is desirable for the duration of the product in the oral cavity, as well as the rate at which the active composition is released from the oral composition, to be controlled so as to optimize the effectiveness of the product in combating oral malodor caused by tobacco smoke. For example, in the case of a chewing gum, administration typically comprises chewing of the gum for at least 5 minutes, more typically for 5 to 60 minutes, even more typically for 10 to 20 minutes and, still more typically, for about 20 minutes. In the case of a chewing gum, typically at least 5%, at least 10%, at least 25%, at least 50%, at least 75%, or even about 100% of the active composition is released from the gum during the first few minutes (e.g., the first about 2 minutes, about 3 minutes, about 4 minutes, or even about 5 minutes) of chewing, More typically, at least 25%, at least 50%, at least 75%, or even at least 100% of the active composition is released from the gum during the first 20 minutes of chewing.

It is to be noted in this regard, however, that in various alternative embodiments, a more sustained delivery of active composition into the oral cavity may be desired. Thus, in such embodiments it may be desired for no more than 25%, no more than 50%, or no more than 75% of the active composition to release into the oral cavity during the first few minutes (e.g., the first about 2 minutes, about 3 minutes, about 4 minutes, or even about 5 minutes) of administration. Likewise, it may be desired for no more than 50% or no more than 75% of the active composition to release into the oral cavity during the first 20 minutes of administration.

### Method for Promoting the Use of a Composition

The present invention is also directed to a method for promoting an oral composition containing a composition effective to reduce the concentration of a pyridine or pyrazine compound present in a subject's oral cavity as a result of smoking a tobacco product. Generally, this process comprises distributing, to an end user or alternatively to someone who will in turn distribute to an end user, an oral composition including, but not limited to, a confection, chewing gum, lozenge, pressed tablet, edible film, mouthspray, mouthwash, or toothpaste product containing a composition recognized to reduce a concentration of a pyridine or pyrazine compound present in a subject's oral cavity as a result of smoking a tobacco product to that subject, and encouraging a subject to consume or chew the product to ameliorate oral malodor resulting from smoking a tobacco product. For example, this encouragement may typically appear on a package containing a confection or chewing gum product and may be disseminated by conventional means (e.g., electronic or print media). Generally, the product is described as containing an ingredient recognized to reduce a concentration of a pyridine or pyrazine compound present in a subject's oral cavity as a result of smoking a tobacco product. In particular, the recognition of the ingredient's effectiveness is achieved by carrying out the screening method described herein. In the case of a chewing gum containing such an ingredient, generally the subject is encouraged to chew the gum for a certain period of time (e.g., at least 5 minutes or about 20 minutes).

The present invention is further illustrated by the following Examples. These Examples are not to be regarded as limiting the scope of the invention or the manner in which it may be practiced.

### EXAMPLES

### Example 1

This example details identification of the odorous components responsible for oral malodor caused by smoking a tobacco product.

The method for identification of the odorous components included swabbing the oral cavity of a subject prior to and after smoking a tobacco product and analysis utilizing solid phase microextraction (SPME), gas chromatography-olfactometry (GCO) and gas chromatography/mass spectroscopy (GC/MS).

Testing for the odorous compounds was conducted at the Microanalytics lab (Microanalytics, A MOCON company Round Rock, TX) and utilized Macanudo cigars (Macanudo^{®} robusto brand, Dominican Republic) using two panelists. The panelists did not eat or drink or use oral hygiene products for 2 hours prior to smoking. Panelists refrained from smoking or consuming any foodstuff except water one hour prior to smoking. Neither panelist regularly smoked, and panelists both possessed normal oral health. Each panelist smoked one-half of a cigar (approximately a 20 minute smoke).

Odorous tobacco smoke compounds were extracted from the panelists' oral cavities by swabbing the tongue's surface fore and aft, five strokes, with a nylon stemmed, nylon mesh coated swab (TX 714A, The Texwipe Co., Upper Saddle River, NJ). After verifying the swab possessed tobacco smoke odor by sensory screening (2 judges sniffed swabs and agreed on most odorous swabs), the polypropylene stem was cut off, and the swab head was sealed in a 40 ml glass vial with a plastic screw cap and Teflon septa for subsequent headspace sampling. Blank swabs taken from the panelists' oral cavities 10 minutes before smoking were analyzed as controls.

Headspace of the vial containing the swab head was extracted for 60 minutes utilizing solid phase microextraction (SPME) (Supelco, Bellefonte, PA) with a Carboxen - polydimethyl siloxane fiber (75 µm, 23 gauge).

The SPME fiber assembly was injected into an Agilent 6890 gas chromatograph (GC) / mass spectrometer (MS) modified for multidimensional analyses (Agilent Technologies, Palo Alto, CA) and equipped with a sniff port and Aroma Trax software (Microanalytics, Round Rock, TX) for the analyses. Fibers remained in the GC injection port for five minutes following injection. Initially, manual SPME extractions were conducted for analysis.

The GC/MS operating parameters included a He carrier gas flow rate of 6.5 ml/min, split mode (2:1) and the injector set at 250°C. Column 1 was 15 meter, 0.53 mm I.D, film thickness 1 µm with 5% phenyl methylpolysiloxane stationary phase (SGE BP5) and was operated with constant pressure mode at 16 psi at an average velocity 66 cm/sec. Column 2 was a 30 meter, 0.53 mm wax capillary with column film thickness 1 µm (SGE BP20) and was operated at a pressure of 5.7 psi at an average velocity 56 cm/sec. The oven was programmed to hold at 40°C for 3 minutes and then increase at 7°C/min to 220°C and hold for 20 minutes. The MS operated in Electron Impact Mode (E.I.) at 70 eV.

The GC sniff port was used to identify specific times of column eluant that exhibited odor characteristic of tobacco smoke; heartcuts (small segments) of chromatographic effluent that contained these odor peaks were selectively analyzed by the MS detector and sniff port for further evaluation and identification. The heart-cut valve was located between the first column and the second column. Second column eluant was split between the MS detector and sniff port (50:50) whereas eluant from the first column traveled exclusively to the flame ionization detector (FID) unless selectively sent to the second column by the heart-cutting valve or unless purged.

Additional testing and further refinement of aroma active compound identities were conducted at the Wrigley Chicago Research and Development facility using a Microanalytics GC/MS unit with identical features as that utilized previously, with the exception that the Chicago unit contained a Leap Technologies CombiPal autosampler capable of utilizing automated SPME (Leap Technologies, Carrboro, NC). The GC/MS unit also had a direct connection between the output of the first column and the sniff port to make heart cutting more accurate and precise. In the previous analysis utilizing manual sampling, the sniff port was connected to the output of the second column only. The time utilized for heart-cuts was based on back-calculating when an odorous peak with a specific retention time at the MS and at the sniff port (at the output of the second column) had transited the first column (the heart cut valve was located on the output of the first column). In this instrument incorporating the autosampler the sniff port had plumbing that allowed for direct connection to the output of the first column, thus heart-cuts could be made based on odor detection time. The sniff port also had the option of connection to the output of the second column as before.

For components that exhibited odor activity but the identity of the component could not be firmly established, the heart-cut effluent was cryogenically focused onto the head of the second column (utilizing a feature of this instrument that contained a spray nozzle that utilized liquid CO₂) to provide additional peak separation. Headspace extraction times utilizing SPME were also extended up to 24 hours to fully load the fiber with headspace volatiles. Other than specified, columns, oven, and other analytical parameters remained the same as previously discussed.

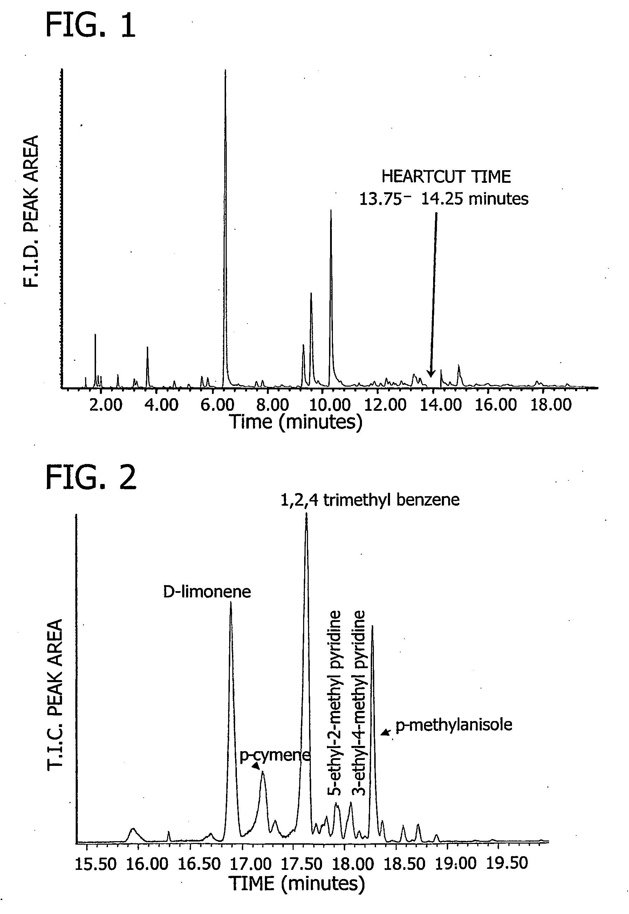

Fig. 1 is an FID chromatogram showing extracted tobacco smoke components of a heart cut taken from 13.75 minutes to 14.25 minutes after injection of the sample in the chromatograph.

Fig. 2 is a total ion current (TIC) chromatogram TIC of heart cut effluent from the output of column 1 from 13.75 to 14.25 minutes after injection of the sample in the chromatograph.

Identification of the odorous components responsible for tobacco breath was also performed by entrapment of smoke volatiles in simulated saliva followed by SPME, GCO and/or GC/MS analysis. Simulated saliva was prepared in accordance with an in-house method by dissolving sodium chloride, sodium bicarbonate and potassium bicarbonate in deionized water to produce a solution with electrolytes present in concentrations similar to saliva. This solution was utilized to measure residual tobacco smoke components following exposure in a manner similar to saliva exposure in the mouth. To prepare saliva-like solutions exposed to cigar smoke, a panelist drew smoke from a cigar (Onyx brand; Dominican Republic, mini Belicoso) and then gently blew four separate two second puffs with a standard drinking straw into the bottom of a 22.5 ml vial so that smoke bubbled through the solution. Liquid contents were then transferred to a separate vial to ensure that any smoke volatiles adsorbed on the glass surfaces were excluded.

Two panelists evaluated SPME extracts of the solution by gas chromatography olfactometry (GCO) analysis three times each and only peaks with the strongest odor intensities were further analyzed by heart cutting. The additional GCO analyses provided greater accuracy and precision than obtained previously. Confirmation of peak identities were made by comparing retention times of identified compounds with those of standard compounds and by comparing odors of identified components with standards.

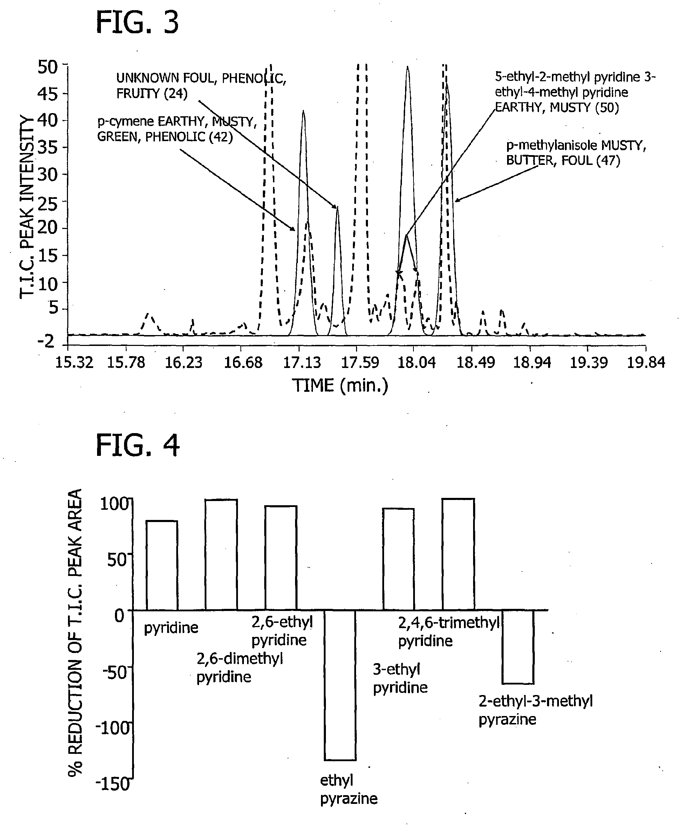

Fig. 3 is an overlay of aromagram and TIC analysis of effluent from SPME headspace extraction of artificial saliva (23 hours extraction time at room temperature) followed by heart-cutting (30 second heart-cut) and cryogenic focusing (30 seconds before, during, and 30 seconds after the heart-cut) with odor intensity score and odor character displayed.

Compounds that possessed aroma activity in initial analyses conducted at Microanalytics were classified as Tier one, two or three. Those in Tier one exhibited the greatest aroma. In subsequent tests conducted at the Wrigley R&D facility, aroma active compounds were assigned numerical scores in accordance with aroma intensity (Table 1).

Identification of components was conducted by matching spectra utilizing a Wiley database (e.g., Wiley Registry of Mass Spectral Data, 7th Edition, John Wiley & Sons, Inc., 2000), and by matching retention indices with authentic standards. Results are shown in Table 1.

**Table 1**

| Preliminary identification of compounds responsible for the odor of tobacco smoke extracted from the oral cavity. | | | |
|---|---|---|---|
| Odor intensity (0-100) | Retention Indice (Kovats) | Descriptor | Identity |
| 67¹ | 1046 | Tobacco, musty | 2,3,5-trimethylpyridine ^{3,4} |
| 45¹ | 945 | Tobacco, earthy | 4-ethyl-3-methyl pyridine ^{3,4} |
| 50¹ | 967 | Nutty | 2-ethyl-3,5-dimethyl pyridine ^{3,4} |
| 58¹ | 844 | Savory | 2,5-dimethyl pyrazine ^{3,4} |
| 47¹ | 972 | Nutty | 2,6-diethyl pyrazine ³ |
| 45¹ | 1034 | Tobacco, musty | 2,4,6-trimethyl pyridine (collidine)^{3,4} and 2-ethyl-5-methyl pyridine ¹ |
| 45¹ | 401 | Floral | Acetophenone ³ |
| Tier 1² | 979 | Musty | ethyl pyrrole ³ |
| Tier 2² | 856 | Savory | 2,3-dimethyl pyrazine ^{3,4} |
| Tier 3² | 830 | Nutty | methyl pyridine ³ |
| Tier 3² | 945 | Tobacco | cyclobutyl pyridine ³ |
| Tier 3² | 525 | Buttery | Diacetyl ³ |

| | | | |
|---|---|---|---|
| ¹ Aroma active compounds measured utilizing simulated saliva blend. ² Aroma activity of compounds measured utilizing swabs and assigned position in 1st, 2nd, or 3rd Tier, according to aroma intensity, only. ³ Compounds identified by correlation with Wiley mass spectrometry database. ⁴ Compounds identified by matching retention indices of authentic standard. | | | |

### Comparative Example 2

This example details *in vitro* GC/MS headspace and sensory measurement of Applephenon^{®} (a natural apple extract powder high in short chained polyphenols and highly soluble in water; A.M. Todd Co. Kalamazoo, MI distributors of Applephenon^{®} for Asahi Corp., Japan) vs. tobacco smoke odor in model solutions.

Measurement of the relationship between tobacco odorant headspace concentration and mass of active added (dose response) included preparation of a model solution composed of compounds with a similar combined odor character and intensity as that in the oral cavity following consumption of two Marlboro light cigarettes, or one-half robust cigar (such as Partagas 1845). These compounds were placed in a 1% ethanol/99% water solution, each at a concentration of 150 ppm (µg/ml solution) and included: collidine (2,4,6-trimethyl pyridine), lutidine (2,6-dimethyl pyridine), 2-ethyl pyridine, 3-ethyl pyridine, pyridine, ethyl pyrazine, 2-ethyl-3-methyl pyrazine.

In order for a substance to possess aroma, it typically passes through the nasal epithelium, via retronasal or orthonasal entrance. Saliva is 99% water and near pH 7 (actual pH depends on salivary flow rate). For this reason the tobacco smoke odor model solution containing 99% water was utilized for initial testing for active efficacy. By reducing headspace concentrations of the odors from aqueous solution, less aroma perception via retro or orthonasal means may result.

The similarity between odor character and intensity of the model solution and oral cavity tobacco smoke malodor was determined by ten R&D personnel experienced in sensory analysis. Odor judges smelled 22.5 ml vials with 5 ml of aqueous solution and assessed perceived aroma character and intensity.

A model solution (5 ml) was treated with the potential ameliorating active, Applephenon^{®} (50 mg wt/wt). Headspace of the resulting aqueous solution was extracted in 22 ml vials with Teflon septa for 10 min. at 35°C with solid phase microextraction fiber (Stabilflex, Carboxen, PDMS, DVB) and analyzed using GC/MS and a CombiPal autosampler as described above in Example 1. Three replications were assessed for % relative standard deviation (RSD). Values in excess of 15% were repeated. Percentage of headspace reduction was assessed by comparison of headspace values of standard with no added actives. The results are shown in Fig. 4. As shown, all pyridine headspace concentrations were reduced by more than 75%.

The aqueous solution with pyrazines/pyridines had a pH 7. Following addition of polyphenol, the solution was pH 3.7 (the drop in pH is attributed to a major component of Applephenon^{®}, the acidic polyphenol chlorogenic acid). Pyridine's pKa is 5.2, whereas pyrazine's is 1.1. In the solution with added polyphenol, pyridine existed primarily as a cation and pyrazine remained neutral. As a charged species, hydrogen bonding in the aqueous solution could account for the decreased volatility associated with pyridine in its protonated form. It was hypothesized the partition coefficient for pyrazine, under acidic aqueous conditions, favored the gaseous state thus the increased headspace concentration.

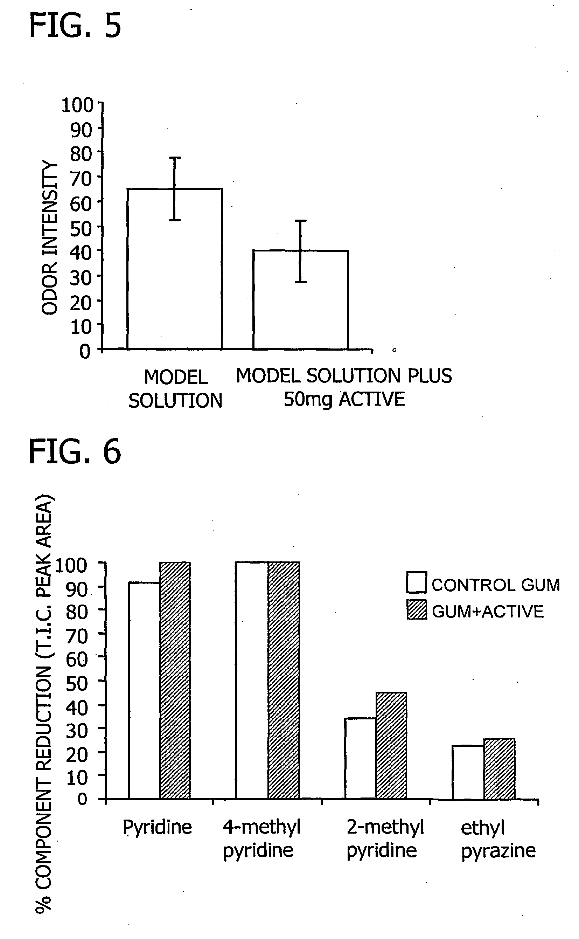

Sensory analysis of identical solutions was conducted with twenty panelists who evaluated the odor intensity of a model solution with and without added active. Odor intensity was assessed utilizing a 0-100 point scale ballot with 0 = no odor and 100 = very strong odor. Responses were averaged and analysis of variance (ANOVA) conducted to assess statistical significance. The results from sensory analysis of identical (but separate) solutions of odorants previously utilized for analytical testing are listed in Fig. 5. Panelists (N=10) found the solution with added polyphenol was significantly lower in tobacco odor intensity vs. the control with P value < 0.05. This corresponds with the decrease in headspace concentration of nitrogenous components previously discussed.

### Comparative Example 3

Following initial in vitro tests that indicated potential active efficacy vs. tobacco odor, a preliminary test was designed to measure changes in residual tobacco smoke compounds present on the tongue and extracted with a swab as previously described.

Evaluation of active efficacy utilizing swabs by smoking one-half of an Onyx cigar and removing a sample of volatiles from the tongue with a Texwipe swab in the manner previously described, the effects of three treatments (Orbit^{®} apple chewing gum commercially available from the Wm. Wrigley Jr. Company (Chicago, IL) plus 78 mg Applephenon^{®}, Orbit^{®} apple gum, and no gum) on residual oral cavity tobacco odor were evaluated by GC/MS. Gums were chewed for 20 minutes immediately following smoking. Swabs generated by two panelists were evaluated. Testing was held in late morning. Panelists consumed a light breakfast 2 hours before testing. Panelists were not cigarette smokers, and had no abnormal oral health problems.

Headspace analyses of panelist swabs before and after treatment with a cigar + no gum, cigar + control gum, and cigar + gum with active and no cigar base line were conducted. The results are shown in Fig. 6. The gum containing the active and the control gum dramatically reduced levels of pyridine (100% and 91% reduction, respectively), and 4-methyl pyridine (both gums reduced levels to below the detection limit) below the level found in the oral cavity following smoking a cigar and then not chewing any gum. Levels of 2-methyl pyridine were reduced 45% by gum with active and 35% by gum without active. Ethyl pyrazine levels were reduced approximately the same by both gums (28% and 25%). Swabs obtained from oral cavities not exposed to cigar smoke did not possess any of the pyridines, but did possess low levels of ethyl pyrazine.

Because model solution headspace data analyzed in vitro (as previously explained) exhibited increased levels of pyrazines following treatment with the active, a reduction of ethyl pyrazine in the samples obtained following chewing gum + active below the level of that from the control gum was not expected and not observed. In general, differences between trace pyridine levels extracted from cigar + control gum and cigar + gum with active were negligible. This negligible difference between control and active gums may be attributed to the difficulty of extracting pyridines adsorbed to the surface of the tongue and then accurately measuring their concentration. Also, with two panelists statistical analyses were not meaningful. Nevertheless, the effect of chewing gum, either active or control, is readily evident in the decrease of odorant headspace concentrations.

### Comparative Example 4

This example details a clinical assessment to determine if Orbit^{®} Apple gum with added active (Applephenon^{®}) reduced self-perceived malodor intensity caused by cigarettes as compared to a control gum without the active and using no gum at all.

A group of one hundred and two panelists were recruited by Tragon Corp. from the Chicago, IL area (Tragon, Buffalo Grove, IL) to smoke two Marlboro light cigarettes and then rate their tobacco aftertaste intensities at time 0 (immediately following smoking), and then at the following times in minutes following smoking: 1, 5, 10, 15, 20, 21, 25, 30, 35, 50, 60, 80 and 90. Three treatments (three sample complete block design) were utilized following smoking (each panelist received a different treatment on three consecutive days and treatments were administered in random order). Test protocol included the following: consumers were asked not to eat, drink, smoke or use oral hygiene (includes brushing and mouthwash) 90 minutes prior to test. Each consumer evaluated all samples and treatments over 6 days; 1 sample per day. Sessions lasted two hours and each session accommodated 25-30 consumers. Four sessions per day were held. Consumers were selected based on smoking habits, all were gum chewers, all possessed no dental or other confounding health problems. Consumers did not consume food or beverage or use oral hygiene for 2 hours before each session. Consumers were given fifteen minutes or less to smoke two cigarettes. Consumers smoked in a designated area outside the building and immediately after smoking, consumers returned inside and rated intensity of tobacco aftertaste using a line scale for intensity.

Treatment one was chewing a serving of pellet gum (serving equal 2 pellets) with the active mixed into gum center (Orbit^{®} Apple plus Applephenon^{®}) for 20 minutes following smoking. Treatment two was chewing a control gum made in the identical manner as before, only with no active, and treatment three was no gum (panelists simply smoked the cigarettes and rated their aftertaste intensities at the appointed times). Panelists received no training on rating tobacco aftertaste and were simply asked to rate their aftertaste intensity based on the method they normally employ to discern if their breath is fresh. A repeated measures Analysis of Covariance was performed on the intensity data from all time points and a one tailed test was employed to determine level of significance.

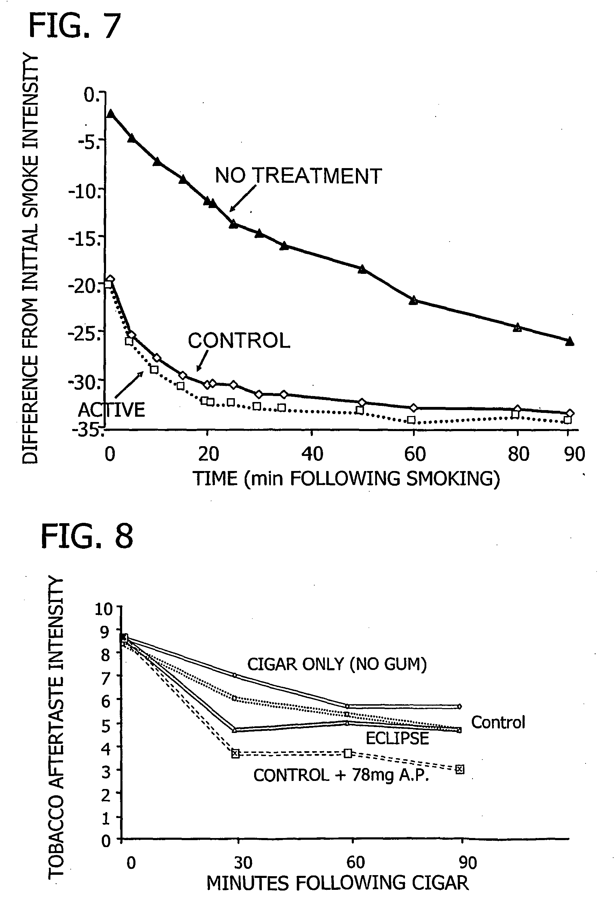

The effect of added active (Applephenon^{®}, 78mg) on perceived tobacco smoke aftertaste associated with smoking two Marlboro light cigarettes is shown in Fig. 7. Results indicated aftertaste intensities determined by a consensus of 102 untrained panelists after chewing Orbit^{®} apple gum treated with 78 mg active was lower than the control at all time points and significantly lower (P < 0.05) at times 20, 21, and 25. Additionally the gum treated with active had a significantly lower mean curve height than the control (P < 0.05). Both active and control gums were judged significantly better than chewing no gum at all vs. cigar aftertaste for all time points.

### Example 5

This example describes a clinical assessment to measure efficacy of Wrigley's Orbit^{®} Apple Gum with added active (Applephenon^{®}) for reducing self-perceived breath malodor intensity caused by smoking a cigar.

Seven panelists were trained in five separate smoking sessions. In each session Onyx cigars were smoked in the manner previously described. After finishing one-half of the cigar panelists rated their self-perceived breath malodor intensity utilizing a 0-10 point line scale at identical times as described with cigarettes. Testing was conducted over four days (two days per week for two consecutive weeks). Four separate treatments (one treatment per day for four consecutive days) were evaluated over the 90 minute interval in random order and included no treatment, one serving of Eclipse Winterfresh^{™} gum commercially available from the Wm. Wrigley Jr. Company (Chicago, IL), one serving of Wrigley's Orbit^{®} Apple gum, one serving of a Wrigley's Orbit^{®} Apple gum with added active (Applephenon^{®}). Following smoking, an initial self-perceived breath malodor assessment was made followed by 20 minutes of gum chewing. After expectoration, self perceived odor assessments were made at 30 minutes, 60 minutes and 90 minutes.

Preliminary testing by measuring self-perception of aftertaste following smoking cigars indicated potential active efficacy. The results are shown in Fig. 8. Results indicated aftertaste intensity was reduced the most, and remained the lowest following chewing Orbit^{®} apple flavored gum with 78 mg of active (Applephenon^{®}). Aftertaste intensity scores were significantly lower than those obtained following chewing the control gum for each time period (P < 0.05). Additionally, aftertaste intensity scores obtained following chewing the gum with 78 mg active were significantly lower than scores measured after chewing Eclipse Winterfresh^{™} gum at times 60 min. and 90 min. Lastly, chewing gum, any gum, significantly reduced cigar aftertaste intensity vs. chewing no gum at all for each time period.

### Example 6

A model solution containing 150 ppm (µg/ml) of each of pyridine, 2-ethyl pyridine, 3-ethyl pyridine and ethyl pyrazine, added to a 5% ethanol, 95% water solution was determined by consensus of odor judges to best represent tobacco smoke odor character and intensity.

A model solution (5 ml) was treated with each of two potential ameliorating actives in separate vials. The first potential active was cranberry extract, a highly concentrated natural cranberry extract powder soluble in water (Ocean Spray Corp., Winthrop, MA); analyses were conducted utilizing 50 mg, 100 mg, 150 mg, and 200 mg of the active. The second potential active was cardamom oil, a natural extract from crushed cardamom seed (Treatt Flavors, Lakeland, FL); analyses were conducted utilizing the potential active in the odorous solution at the following levels: 54 µg/ml, 156 µg/ml, 207 µg/ml and 642 µg/ml.

Headspace of the aqueous solution was extracted in 22 ml vials with Teflon septa for 10 min at 35°C using a solid phase microextraction fiber (Stabilflex, Carboxen, PDMS, DVB) and analyzed with GC/MS. A CombiPal autosampler was utilized as described above. Three replications were assessed for % relative standard deviation (RSD). Values in excess of 15% were repeated. Percentage of headspace reduction was assessed by comparison of headspace values of a standard with no added actives.

In order for a substance to possess aroma, it typically is volatile and passes through the nasal epithelium, via retronasal or orthonasal entrance. Saliva is 99% water and near pH 7 (actual pH depends on salivary flow rate). For this reason the tobacco smoke odor model solution containing 99% water was utilized for initial testing for active efficacy. By reducing headspace concentrations of the odors from aqueous solution, less aroma perception via retro or orthonasal means may result.

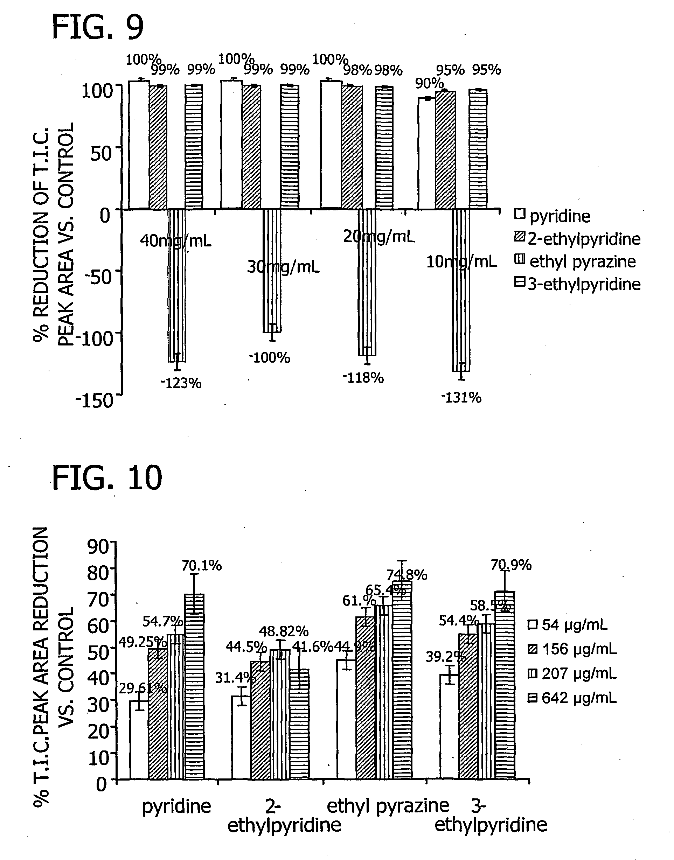

Fig. 9 shows the results from the addition of 50 mg, 100 mg, 150 mg and 200 mg of cranberry extract. Tables 2-5 show the analytical dose response data for headspace concentration reduction associated with pyridine, 2-ethyl pyridine, ethyl pyrazine and 3-ethyl pyridine, respectively.

**Table 2**

| Cranberry extract reduction of pyridine headspace concentration (expressed as mg cranberry per ml solution): analytical dose response data. | | | | | |
|---|---|---|---|---|---|
| | GC/MS Peak Area | | | | |
| | Control | 40 mg/ml | 30 mg/ml | 20 mg/ml | 10 mg/ml |
| Run 1 | 14444526 | n.d.¹ | n.d.¹ | n.d.¹ | 1558183 |
| Run 2 | 14571161 | n.d.¹ | n.d.¹ | n.d.¹ | 1572148 |
| Run 3 | 16085301 | n.d.¹ | n.d.¹ | n.d.¹ | 1579508 |
| Mean | 15033663 | n.d.¹ | n.d.¹ | n.d.¹ | 1569946 |
| Standard Deviation | 912943.9 | n.d.¹ | n.d.¹ | n.d.¹ | 10831.64 |
| Standard Error | 527088.4 | n.d.¹ | n.d.¹ | n.d.¹ | 6253.65 |
| %RSD | 6.07 | n.d.¹ | n.d.¹ | n.d.¹ | 0.69 |

| | | | | | |
|---|---|---|---|---|---|
| ¹ n.d. indicates that pyridine was not detected | | | | | |

**Table 3**

| Cranberry extract reduction of 2-ethylpyridine headspace concentration (expressed as mg cranberry per ml solution) : analytical dose response data. | | | | | |
|---|---|---|---|---|---|
| | GC/MS Peak Area | | | | |
| | Control | 40 mg/ml | 30 mg/ml | 20 mg/ml | 10 mg/ml |
| Run 1 | 160109870 | 1368141 | 1645724 | 1948092 | 6876637 |
| Run 2 | 142768839 | 1496262 | 1565780 | 2264499 | 6660330 |
| Run 3 | 173862114 | 1538198 | 1372219 | 1913381 | 6356517 |
| Mean | 158913607.7 | 1467533.6 | 1527907.6 | 2041990.6 | 6631161.3 |
| Standard Deviation | 15581117.4 | 88593.6 | 140630.6 | 193477.8 | 261283.9 |
| Standard Error | 8995762.3 | 51149.5 | 81193.1 | 111704.4 | 150852.3 |
| % RSD | 9.81 | 6.04 | 9.19 | 9.47 | 3.94 |

**Table 4**

| Cranberry extract reduction of ethyl pyrazine headspace concentration (expressed as mg cranberry per ml solution): analytical dose response data. | | | | | |
|---|---|---|---|---|---|
| | GC/MS Peak Area | | | | |
| | Control | 40 mg/mL | 30 mg/mL | 20 mg/mL | 10 mg/mL |
| Run 1 | 35558030 | 76470454 | 71101842 | 73272062 | 82832901 |
| Run 2 | 31179441 | 77280290 | 68358517 | 78616280 | 80690737 |
| Run 3 | 36276405 | 76581435 | 66278482 | 73562475 | 74884246 |
| Mean | 34337959 | 76777393 | 68579614 | 75150272 | 79469295 |
| Standard Deviation | 2758839 | 439042.4 | 2419269 | 3005161 | 4112690 |
| Standard Error | 1592816 | 253481.3 | 1396766 | 1735030 | 2374463 |
| % RSD | 8.03 | 0.57 | 3.53 | 4.01 | 5.18 |

**Table 5**

| Cranberry extract reduction of 3-ethylpyridine headspace concentration (expressed as mg cranberry per ml solution): analytical dose response data. | | | | | |
|---|---|---|---|---|---|
| | GC/MS Peak Area | | | | |
| | Control | 40 mg/ml | 30 mg/ml | 20 mg/ml | 10 mg/ml |
| Run 1 | 1.81 x 10⁸ | 1096887 | 1365358 | 2109647 | 7721021 |
| Run 2 | 1.61 x 10⁸ | 1361864 | 1130701 | 2107292 | 7025718 |
| Run 3 | 1.86 x 10⁸ | 1201307 | 1193170 | 2357495 | 7174169 |
| Mean | 1.76 x 10⁸ | 1220019 | 1229743 | 2191478 | 7306969 |
| Standard Deviation | 13293496 | 133475.9 | 121528.5 | 143779.8 | 366181 |
| Standard Error | 7675003 | 77062.35 | 70164.49 | 83011.28 | 211414.7 |
| % RSD | 7.55 | 10.94 | 9.88 | 6.56 | 5.01 |

Fig. 10 shows the results from addition of 54 µg/ml, 156 µg/ml, 207 µg/ml and 642 µg/ml levels of cardamom oil to the model solution containing pyridine, 2-ethyl pyridine, 3-ethyl pyridine and ethyl pyrazine. Tables 6-10 show the analytical dose response data for headspace concentration reduction associated with pyridine, 2-ethyl pyridine, ethyl pyrazine and 3-ethyl pyridine, respectively.

**Table 6**

| Cardamom oil reduction of pyridine headspace concentration (expressed as µg cardamom oil per ml solution): analytical dose response data. | | | | | |
|---|---|---|---|---|---|
| | GC/MS Peak Area | | | | |
| | Control | 642 µg/ml | 207 µg/ml | 156 µg/ml | 54 µg/ml |
| Run 1 | 15992341 | 4784811 | 6911030 | 7410541 | 10584810 |
| Run 2 | 15965030 | 4659265 | 7052210 | 7928636 | 11057573 |
| Run 3 | 15177671 | 4650976 | 7387659 | 8581892 | 11536473 |
| Mean | 15711680.6 | 4698350.6 | 7116966.3 | 7973689.6 | 11059618.6 |
| Standard Deviation | 462667.5 | 74991.4 | 244824.0 | 586973.7 | 475834.7 |
| Standard Error | 267121.2 | 43296.3 | 141349.2 | 338889.4 | 274723.3 |
| % RSD | 2.94 | 1.59 | 3.44 | 7.36 | 4.31 |

**Table 7**

| Cardamom oil reduction of 2-ethylpyridine headspace concentration (expressed as µg cardamom oil per ml solution): analytical dose response data. | | | | | |
|---|---|---|---|---|---|
| | GC/MS Peak Area | | | | |
| | Control | 642 µg/ml | 207 µg/ml | 156 µg/ml | 54 µg/ml |
| Run 1 | 184268848 | 112408826 | 94359048 | 97679714 | 122375677 |
| Run 2 | 182003143 | 105790353 | 91684882 | 100144739 | 122903012 |
| Run 3 | 173676457 | 97123074 | 90300102 | 101340819 | 124912319 |
| Mean | 179982816 | 105107417.7 | 92114677.3 | 99721757.3 | 123397002.7 |
| Standard Deviation | 5577721.5 | 7665725.9 | 2063323.4 | 1866844.2 | 1338528.3 |
| Standard Error | 3220299.0 | 4425808.9 | 1191260.3 | 1077823.0 | 772799.7 |
| % RSD | 3.09 | 7.29 | 2.24 | 1.87 | 1.08 |

**Table 8**

| Cardamom oil reduction of ethylpyrazine headspace concentration (expressed as µg cardamom oil per ml solution): analytical dose response data. | | | | | |
|---|---|---|---|---|---|
| | GC/MS Peak Area | | | | |
| | Control | 642 µg/ml | 207 µg/ml | 156 µg/ml | 54 µg/ml |
| Run 1 | 40331343 | 9935627 | 12937889 | 13960096 | 20136295 |
| Run 2 | 38850725 | 9687259 | 13204900 | 15240288 | 21393217 |
| Run 3 | 36946768 | 9619715 | 13946772 | 16060245 | 22360332 |
| Mean | 38709612 | 9747533.6 | 13363187 | 15086876.3 | 21296614.6 |
| Standard Deviation | 1696694.32 | 166357.6 | 522735.4 | 1058445.9 | 1115161.0 |
| Standard Error | 979586.92 | 96046.6 | 301801.4 | 611094.0 | 643838.5 |
| % RSD | 4.38 | 1.71 | 3.91 | 7.02 | 5.24 |

**Table 9**

| Cardamom oil reduction of 3-ethylpyridine headspace concentration (expressed as µg cardamom oil per ml solution): analytical dose response data. | | | | | |
|---|---|---|---|---|---|
| | GC/MS Peak Area | | | | |
| | Control | 642 µg/ml | 207 µg/ml | 156 µg/ml | 54 µg/ml |
| Run 1 | 185543821 | 52981160 | 70792235 | 75103881 | 103474622 |
| Run 2 | 176086450 | 50707967 | 73101477 | 80706289 | 107019840 |
| Run 3 | 168268511 | 50069965 | 75972042 | 85414846 | 111211778 |
| Mean | 176632927.3 | 51253030.6 | 73288584.6 | 80408338.6 | 107235413.3 |
| Standard Deviation | 8650610.4 | 1530223.8 | 2594967.6 | 5161935.7 | 3873080.1 |
| Standard Error | 4994432.3 | 883475.1 | 1498205.2 | 2980244.9 | 2236123.8 |
| % RSD | 4.89 | 2.98 | 3.54 | 6.42 | 3.61 |

With cranberry extract, all pyridine headspace concentrations were reduced by more than 85%. Rather than a decrease in headspace concentrations, pyrazine levels.actually increased; this effect is believed to be attributed to the pH of the solution containing cranberry extract.

Sensory analyses of the model solutions containing cranberry extract and cardamom oil were conducted by ten panelists who evaluated the odor intensity of tobacco odorant model solutions with and without added active. Odor intensity was assessed utilizing a 0-10 point scale ballot with 0 = no odor and 10 = very strong odor. Responses were averaged and analysis of variance (ANOVA) conducted to assess statistical significance.

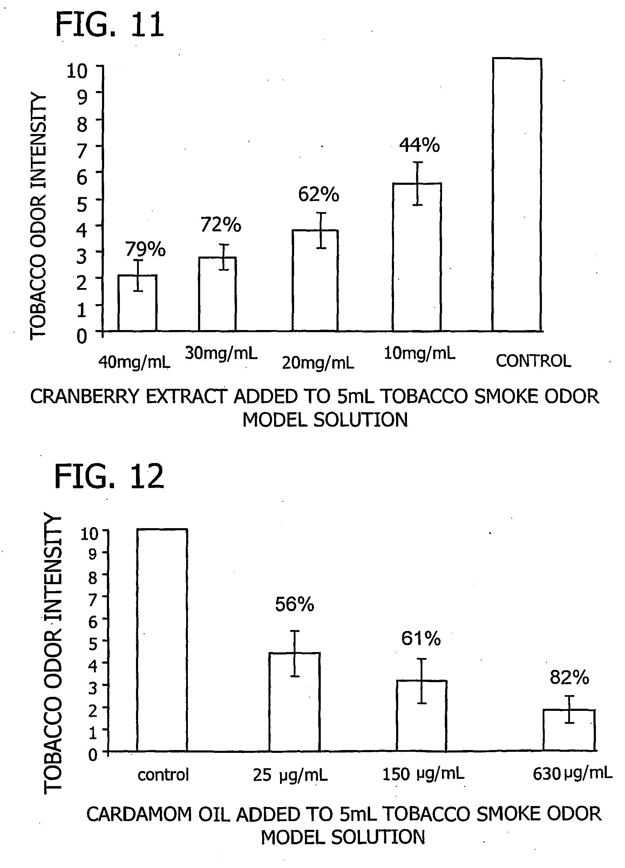

Fig. 11 and Table 10 show the panelist responses at the various amounts of cranberry extract active.

**Table 10**

| Panelist responses (N=10) in rating aroma intensities of tobacco smoke odor solution (pyridines and pyrazines in water) treated with different concentrations of cranberry extract (expressed as mg cranberry/ml model solution) | | | | | |
|---|---|---|---|---|---|
| | Panelist | 40 mg/ml | 30 mg/ml | 20 mg/ml | 10 mg/ml |
| 1 | DP | 3 | 5 | 8 | 7 |
| 2 | DC | 2 | 2 | 5 | 5 |
| 3 | CD | 7 | 5 | 6 | 8 |
| 4 | CM | 3 | 2 | 5 | 10 |
| 5 | DB | 2 | 2 | 3 | 7 |
| 6 | BP | 1 | 3 | 2 | 3 |
| 7 | RB | 1 | 1 | 4 | 5 |
| 8 | MT | 1 | 1 | 1 | 2 |
| 9 | LD | 0 | 5 | 3 | 7 |
| 10 | MD | 1 | 2 | 1 | 2 |
| | Mean | 2.1 | 2.8 | 3.8 | 5.6 |
| | Standard Deviation | 1.868154 | 1.536229 | 2.135416 | 2.537716 |
| | Standard Error | 0.590762 | 0.485798 | 0.675278 | 0.802496 |

Fig. 12 and Table 11 show the panelist responses at the various amounts of cardamom oil active.

**Table 11**

| Panelist responses (N=10) in rating aroma intensities of tobacco smoke odor solution (pyridines and pyrazines in water) treated with different concentrations of cardamom oil (expressed as µg cardamom oil/ml model solution) | | | | |
|---|---|---|---|---|
| | Panelists | 25 µg/ ml | 150 µg/ ml | 630 µg/ ml |
| 1 | JK | 8 | 7 | 3.5 |
| 2 | SM | 1 | 3 | 1 |
| 3 | BM | 5 | 4 | 1 |
| 4 | DB | 9 | 7 | 6 |
| 5 | SM | 8 | 10 | 2 |
| 6 | LD | 5 | 2 | 2 |
| 7 | RB | 0 | 0 | 0 |
| 8 | MT | 3 | 1 | 0 |
| 9 | MH | 1 | 3 | 0 |
| 10 | MD | 4 | 2 | 3 |
| | Mean | 4.4 | 3.9 | 1.85 |
| | Standard Deviation | 3.2 | 3.14 | 1.91 |
| | Standard Error | 1.0 | 0.99 | 0.60 |

As shown, the panelists (N=10) found the solutions with added actives were significantly lower in tobacco odor intensity vs. the control with P value < 0.05.

### Example 7

This example details potential active component release from gum.

The method to measure active release and rate of release involved having 5 panelists chew one serving of gum each (2 pellets), for each of the following times: 0, 10, 20, 25 minutes. Following the chews gum cuds were collected (minimum of 6 cuds), dissolved in a chloroform solvent with undecane as internal standard. The solution was shaken for six hours to ensure solvation. Liquid was then removed and purified with solid phase extraction (SPE) utilizing Millipore (Billerica, MA) Millex-FH hydrophobic PTFE 0.4 µm.

For non-volatile active components, such as those in cranberry extract, the aqueous layer is removed for analysis conducted with high performance liquid chromatography (HPLC). Quinic acid is utilized as the indicator compound from cranberry (the percentage of quinic acid in cranberry is known, therefore by quantitative analysis of quinic acid in the gum, the amount of cranberry in the gum can be ascertained). Other HPLC operating parameters include the following: reversed phase; Restek Allure organic acids column with following dimensions: 5 micron 300 x 4.6 mm. The mobile Phase was 100% 0.1M phosphate buffer, pH of 2.5. Additional conditions: flow = 0.3 ml/min, column temperature was 15°C, run time was 45 minutes, detector wavelength was 210 nm and injection volume was 10 ml.

For volatile components as in cardamom oil, undecane was utilized as an internal standard. The chloroform layer (bottom layer) was removed by Pasteur pipette and placed in a GC vial and capped with crimped cap and Teflon septa. Liquid is injected (in triplicate) into GC for subsequent active component quantification utilizing an Agilent 7683 Series Autosampler (Agilent Technologies, Palo Alto, CA) set to inject 5 µl.

Cranberry extract was placed in the gum center of a standard chewing gum formulation during production. The extract contained 2.3% quinic acid. Results indicated there was 1.5 mg (0.05%) quinic acid in a serving of gum (center plus coating). This equates to 65 mg cranberry in one serving of gum (2 pellets). No quinic acid was detected in gum chewed for 20 minutes, thus all cranberry was released from gum to the saliva during the 20 minute gum chew.

Table 12 summarizes the percent headspace reduction of pyridine and 3-ethyl pyridine and sensory odor intensity reduction. Table 13 summarizes the cranberry active release over the course of a 20 minute chew time.

**Table 12**

| Cranberry summary In-vitro efficacy | | | |
|---|---|---|---|
| Cranberry extract concentration (mg/ml model solution) | % pyridine headspace reduction | % 3-ethyl pyridine headspace reduction | Sensory % odor intensity reduction |
| 10 | 90 | 96 | 44 |
| 20 | 100 | 98 | 62 |
| 30 | 100 | 99 | 72 |
| 40 | 100 | 99 | 79 |

**Table 13**

| Cranberry release from formula | | | | |
|---|---|---|---|---|
| Chew time (min) | % in sample | mg found | % released | mg released |
| Test gum | | | | |
| 0 | 100 | 65 | 0 | 0 |
| 20 | 0 | 0 | 100 | 65 |

As shown, 100% of the cranberry extract, or 65 mg, is released from the gum during chewing for 20 minutes. Based on these results, it is believed that this amount of cranberry extract would reduce headspace levels of pyridines > 90% if added to 5 ml of model solution and would reduce odor intensity of 5 ml of model solution between 44% and 62%.

Cardamom oil was added to the gum coating only of a standard chewing gum formulation. It was not added to the center; α-terpinyl acetate comprised 37% of the cardamom oil.

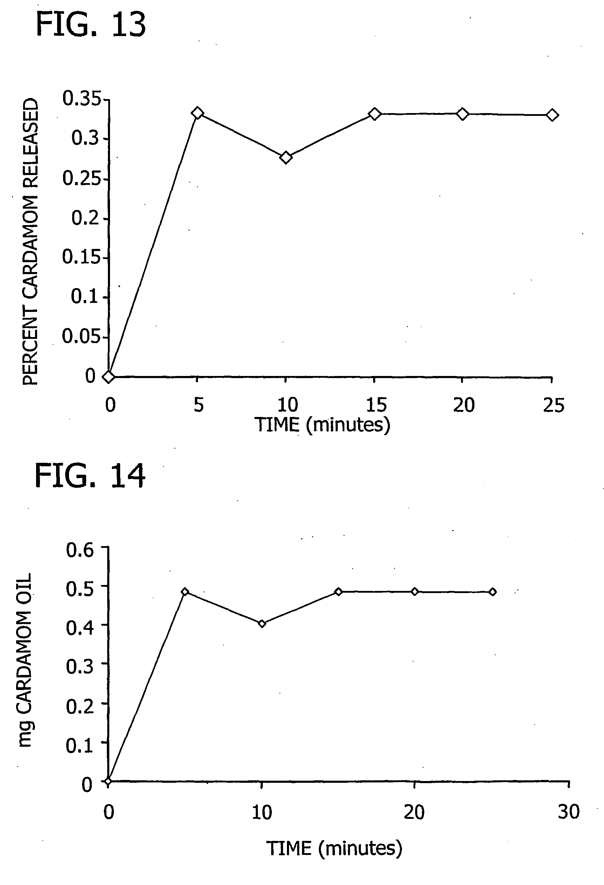

Although d-limonene also was present in cardamom oil in large amount, α-terpinyl acetate was chosen for this experiment because 1,8 cineole could not be separated from d-limonene as per TIC. Cardamom release results utilizing α-terpinyl acetate as the indicator compound are shown in Fig. 13. Cardamom release in terms of milligrams (mg) oil over time are shown in Fig. 14.

The majority of cardamom oil released from gum between 0-5 minutes of chewing (33% decrease in cardamom oil in gum between 0-5 minutes). Table 14 shows the cardamom release over the course of 25 minutes of chewing; as shown, 0.49 mg, or 33% of cardamom oil is released from the gum after chewing.

**Table 14**

| Cardamom release from formula | | | | |
|---|---|---|---|---|
| Chew time (min) | % in sample | mg found | % released | mg released |
| Test gum | | | | |
| 0 | 0.05 | 1.46 | 0 | 0 |
| 5 | 0.03 | 0.97 | 33 | 0.49 |
| 25 | 0.03 | 0.97 | 33 | 0.49 |

Table 15 summarize the percent headspace reduction of pyridine and 3-ethyl pyridine and sensory odor intensity reduction. This value of cardamom release would provide a reduction of approximately >60% of the tobacco smoke odor of 5 ml of model solution. Additionally, this quantity of cardamom oil release would reduce headspace concentrations of all volatiles, including pyrazines, in the model solution >50%.

**Table 15**

| Cardamom summary In-vitro efficacy | | | |
|---|---|---|---|
| Cardamom concentration (µg/ml model solution) | % pyridine headspace reduction | % 3-ethyl pyridine headspace reduction | Sensory % odor intensity reduction |
| 25 | - | - | 56 |
| 54 | 29.6 | 39.2 | - |
| 150 | - | - | 61 |
| 156 | 49.3 | 54.4 | - |
| 207 | 54.7 | 58.5 | - |
| 630 | - | - | 82 |
| 642 | 70.1 | 70.9 | - |

| | | | |
|---|---|---|---|
| - indicates not measured | | | |

The present invention is not limited to the above embodiments and can be variously modified. The above description of the preferred embodiments, including the Examples, is intended only to acquaint others skilled in the art with the invention, its principles, and its practical application so that others skilled in the art may adapt and apply the invention in its numerous forms, as may be best suited to the requirements of a particular use.

With reference to the use of the word(s) comprise or comprises or comprising in this entire specification (including the claims below), unless the context requires otherwise, those words are used on the basis and clear understanding that they are to be interpreted inclusively, rather than exclusively, and applicants intend each of those words to be so interpreted in construing this entire specification.

## Claims

1. A method for identifying a composition suitable for use in an oral composition effective for reducing oral malodour associated with tobacco smoke, the method comprising:
contacting in a vessel a test composition and a model solution comprising tobacco smoke odorants, the odorants comprising pyridine, 2-ethyl pyridine, 3-ethyl pyrazine and ethyl pyrazine, wherein the test composition comprises cardamom oil, rosemary extract, basil extract, thyme extract, parsley seed oil, zinc lactate, eugenol, α-cyclodextrin, β-cyclodextrin, γ-cyclodextrin, activated carbon, or a combination thereof; and
determining the ability of the test composition to reduce the concentration of one or more of the odorants in said model solution in the headspace of said vessel.

2. A method as set forth in claim 1 wherein the model solution further comprises 2,4,6-trimethyl pyridine, 2,6-dimethyl pyridine, 3-ethyl pyridine, 2-ethyl-3-methyl pyrazine, or combinations thereof.

3. A method as set forth in claim 1 or 2 wherein the model solution further comprises 3,5-dimethyl pyridine, 2-ethyl pyridine, 3-ethyl pyridine, 2,3-dimethyl pyrazine, 2,5-dimethyl pyrazine, or combinations thereof.

4. A method as set forth in any one of claims 1 to 3 wherein said oral composition comprises a confection, chewing gum, lozenge, pressed tablet, edible film, mouthspray, mouthwash, toothpaste product or combinations thereof.

5. A method as set forth in any one of claims 1 to 4 wherein the ability of the test composition to reduce the concentration of one or more of the odorants in the headspace of said vessel is determined by a method comprising:
measuring the concentration of one or more of the odorants in the headspace of said vessel containing the model solution prior to contact with the test composition, to determine an initial odorant concentration in said headspace;
measuring the concentration of one or more of the odorants in the headspace of said vessel containing the model solution after contact with the test composition, to determine a final odorant concentration in said headspace; and
determining the difference between the initial and final concentration of one or more of the odorants in the headspace of the vessel.

6. A method as set forth in any one of claims 1 to 5 wherein the concentration of an odorant is reduced by at least 50%.

7. Use of a chewing gum comprising cardamom oil, cranberry extract, or a combination thereof to reduce the concentration of a pyridine or pyrazine compound present in a subject's oral cavity as a result of smoking a tobacco product.

8. Use according to claim 7 wherein the concentration of a pyridine or pyrazine compound is reduced.

9. Use according to claim 8 wherein the concentration of the pyridine or pyrazine compound is reduced by at least 30%.

10. Use according to claim 9 wherein the concentration of the pyridine or pyrazine compound is reduced by at least 50%.

11. Use according to any one of claims 7 to 10 wherein the chewing gum comprises cardamom oil.

## Patentansprüche

1. Verfahren zur Bestimmung einer Zusammensetzung, welche zur Verwendung in einer Oralzusammensetzung geeignet ist, wobei die Oralzusammensetzung wirksam bei der Verminderung von Mundgeruch in Verbindung mit Tabakqualm ist, wobei das Verfahren die folgenden Schritte aufweist:
In Kontaktbringen einer Testzusammensetzung mit einer Tabakqualm-Odoriermittel enthaltenden Musterlösung in einem Behälter, wobei die Odoriermittel Pyridin, 2-Ethylpyridin, 3-Ethylpyrazin sowie Ethylpyrazin aufweisen, **dadurch gekennzeichnet, dass** die Testzusammensetzung Kardamomöl, Rosmarienextrakt, Basilikumextrakt, Thymianextrakt, Petersiliensamenöl, Zinklaktat, Eugenol, α-Cyclodextrin, β-Cyclodextrin, γ-Cyclodextrin, aktivierten Kohlenstoff bzw. Aktivkohle, oder eine Kombination daraus aufweist; und
Bestimmung der Fähigkeit der Testzusammensetzung, die Konzentration eines oder mehrerer Odoriermittel in der Musterlösung in dem Gasraum des Behälters zu verringern.

2. Verfahren nach Anspruch 1, **dadurch gekennzeichnet, dass** die Musterlösung des Weiteren 2,4,6-Trimethylpyridin, 2,6-Dimethylpyridin, 3-Ethylpyridin, 2-Ethyl-3-Methylpyrazin, oder Kombinationen daraus aufweist.

3. Verfahren nach Anspruch 1 oder 2, **dadurch gekennzeichnet, dass** die Musterlösung des Weiteren 3,5-Dimethylpyridin, 2-Ethylpyridin, 3-Ethylpyridin, 2,3-Dimethylpyrazin, 2, 5-Dimethylpyrazin, oder Kombinationen daraus aufweist.

4. Verfahren nach einem der Ansprüche 1 bis, 3, **dadurch gekennzeichnet, dass** die Oralzusammensetzung ein Konfekt, einen Kaugummi, eine Pastille, eine geprägte Tablette, eine essbare Folie, ein Mundspray, eine Mundspülung, ein Zahnpastaprodukt, oder Kombinationen daraus aufweist.

5. Verfahren nach einem der Ansprüche 1 bis 4, **dadurch gekennzeichnet, dass** die Fähigkeit der Testzusammensetzung zur Reduzierung der Konzentration eines oder mehrerer Odoriermittel in dem Gasraum des Behälters durch ein Verfahren bestimmt wird, welches die folgenden Schritte aufweist:
Messung der Konzentration eines oder mehrerer Odoriermittel in dem Gasraum des Behälters, welcher die Musterlösung aufweist, bevor diese mit der Testzusammensetzung in Berührung kommt, um eine anfängliche Odoriermittelkonzentration in dem Gasraum zu bestimmen;
Messung der Konzentration eines oder mehrerer Odoriermittel in dem Gasraum des Behälters, welcher die Musterlösung aufweist, nachdem diese mit der Testzusammensetzung in Berührung gekommen ist, um eine Odoriermittel-Endkonzentration in dem Gasraum zu bestimmen; und
Bestimmung der Differenz zwischen der Anfangs- und Endkonzentration des einen oder der mehreren Odoriermittel in dem Gasraum des Behälters.

6. Verfahren nach einem der Ansprüche 1 bis 5, **dadurch gekennzeichnet, dass** die Konzentration eines Odoriermittels um mindestens 50% verringert wird.

7. Verwendung eines Kaugummis, welcher Kardamomöl, Moosbeerenextrakt, oder eine Kombination daraus aufweist, um die Konzentration einer Pyridin- oder Pyrazin-Verbindung, welche in der Mundhöhle einer Testperson als Ergebnis des Rauchens eines Tabakprodukts vorliegt, zu reduzieren.

8. Verwendung gemäß Anspruch 7, **dadurch gekennzeichnet, dass** die Konzentration einer Pyridin- oder Pyrazin-Verbindung reduziert wird.

9. Verwendung nach Anspruch 8, **dadurch gekennzeichnet, dass** die Konzentration der Pyridin- oder Pyrazin-Verbindung um mindestens 30% verringert wird.

10. Verwendung nach Anspruch 9, **dadurch gekennzeichnet, dass** die Konzentration der Pyridin- oder Pyrazin-Verbindung um mindestens 50% verringert wird.

11. Verwendung nach einem der Ansprüche 7 bis 10, **dadurch gekennzeichnet, dass** der Kaugummi Kardamomöl aufweist.

## Revendications

1. Procédé d'identification d'une composition appropriée pour une utilisation dans une composition buccale efficace pour réduire les mauvaises odeurs buccales associées à la fumée de tabac, le procédé comprenant:
la mise en contact dans une cuve d'une composition d'essai et d'une solution modèle comprenant des éléments odorants de la fumée de tabac, les éléments odorants comprenant la pyridine, la 2-éthyl pyridine, la 3-éthyl pyrazine et l'éthyl pyrazine, dans lequel la composition d'essai comprend de l'huile de cardamome, de l'extrait de romarin, de l'extrait de basilic, de l'extrait de thym, de l'huile de grain de persil, du lactate de zinc, de l'eugénol, de l'α-cyclodextrine, de la β-cyclodextrine, de la γ-cyclodextrine, du charbon actif ou une de leurs combinaisons; et
la détermination de la capacité de la composition d'essai à réduire la concentration d'un ou plusieurs des éléments odorants dans ladite solution modèle dans l'espace de tête de ladite cuve.

2. Procédé selon la revendication 1, dans lequel la solution modèle comprend en outre de la 2,4,6-triméthyl pyridine, de la 2,6-diméthyl pyridine, de la 3-éthyl pyridine, de la 2-éthyl-3-méthyl pyrazine ou leurs combinaisons.

3. Procédé selon la revendication 1 ou 2, dans lequel la solution modèle comprend en outre de la 3,5-diméthyl pyridine, de la 2-éthyl pyridine, de la 3-éthyl pyridine, de la 2,3-diméthyl pyrazine, de la 2,5-diméthyl pyrazine ou leurs combinaisons.

4. Procédé selon l'une quelconque des revendications 1 à 3, dans lequel ladite composition buccale comprend un électuaire, une gomme à mâcher, une tablette, un comprimé, un film comestible, un spray buccal, un bain de bouche, un produit de pâte dentifrice ou leurs combinaisons.

5. Procédé selon l'une quelconque des revendications 1 à 4, dans lequel la capacité de la composition d'essai à réduire la concentration d'un ou plusieurs des éléments odorants dans l'espace de tête de ladite cuve est déterminée par un procédé comprenant:
la mesure de la concentration d'un ou plusieurs des éléments odorants dans l'espace de tête de ladite cuve contenant la solution modèle avant le contact avec la composition d'essai pour déterminer une concentration initiale en éléments odorants dans ledit espace de tête;
la mesure de la concentration d'un ou plusieurs des éléments odorants dans l'espace de tête de ladite cuve contenant la solution modèle après le contact avec la composition d'essai pour déterminer une concentration finale en éléments odorants dans ledit espace de tête; et
la détermination de la différence entre la concentration initiale et la concentration finale d'un ou plusieurs des éléments odorants dans l'espace de tête de la cuve.

6. Procédé selon l'une quelconque des revendications 1 à 5, dans lequel la concentration d'un élément odorant est réduite d'au moins 50 %.

7. Utilisation d'une gomme à mâcher comprenant de l'huile de cardamome, de l'extrait de canneberge ou une de leurs combinaisons pour réduire la concentration d'un composé de pyridine ou de pyrazine présent dans la cavité buccale d'un sujet après avoir fumé un produit de tabac.

8. Utilisation selon la revendication 7, dans laquelle la concentration d'un composé de pyridine ou de pyrazine est réduite.

9. Utilisation selon la revendication 8, dans laquelle la concentration du composé de pyridine ou de pyrazine est réduite d'au moins 30 %.

10. Utilisation selon la revendication 9, dans laquelle la concentration du composé de pyridine ou de pyrazine est réduite d'au moins 50 %.

11. Utilisation selon l'une quelconque des revendications 7 à 10, dans laquelle la gomme à mâcher comprend de l'huile de cardamome.
